# EUROPEAN PATENT SPECIFICATION

(11) **EP 1 883 803 B1**
(45) Date of publication and mention of the grant of the patent: **13.06.2012**
(21) Application number: 06728042.0
(22) Date of filing: 25.04.2006
(51) Int. Cl.: G01N 21/00

(54) **LOW COST APPARATUS FOR DETECTION OF NITROGEN-CONTAINING GAS COMPOUNDS**
KOSTENGÜNSTIGE VORRICHTUNG ZUM NACHWEIS STICKSTOFFHALTIGER GASVERBINDUNGEN
APPAREIL DE FAIBLE COUT POUR DETECTER DES COMPOSES GAZEUX CONTENANT DE L'AZOTE

(30) Priority: 26.04.2005 EP 05300323
(43) Date of publication of application: 06.02.2008
(73) Proprietor: Koninklijke Philips Electronics N.V., 5621 BA Eindhoven (NL)
(72) Inventor: VAN KESTEREN, Hans, Société Civile SPID, F-75008 Paris (FR); VINK, Teunis Johannes, Société Civile SPID, F-75008 Paris (FR); WILLARD, Nicolaas Petrus, Société Civile SPID, F-75008 Paris (FR)
(74) Representative: Schouten, Marcus Maria
(86) International application number: PCT/IB2006/051288
(87) International publication number: WO 2006/114766

(56) References cited:
- WO-A-02/31475
- WO-A2-03/104767
- US-A1- 2004 133 116
- US-B2- 6 873 414
- POLZAT O ET AL: "DETERMINATION OF NITROGEN DIOXIDE BY VISIBLE PHOTOACOUSTIC SPECTROSCOPY" ANAL CHEM AUG 1982, vol. 54, no. 9, August 1982 (1982-08), pages 1485-1489, XP002406407
- FRIED ALAN ET AL: "LASER PHOTOACOUSTIC DETECTION OF NITROGEN DIOXIDE IN THE GAS-PHASE TITRATION OF NITRIC OXIDE WITH OZONE" ANAL CHEM FEB 1982, vol. 54, no. 2, February 1982 (1982-02), pages 278-282, XP002406408
- ROBINSON J K ET AL: "LUMINOL/H2O2 CHEMILUMINESCENCE DETECTOR FOR THE ANALYSIS OF NITRIC OXIDE IN EXHALED BREATH" ANALYTICAL CHEMISTRY, AMERICAN CHEMICAL SOCIETY. COLUMBUS, US, vol. 71, no. 22, 15 November 1999 (1999-11-15), pages 5131-5136, XP000926903 ISSN: 0003-2700
- SANTIAGO G ET AL: "Resonant photoacoustic gas sensing by PC-based audio detection" APPL PHYS B; APPLIED PHYSICS B: LASERS AND OPTICS OCTOBER 2003, vol. 77, no. 4, October 2003 (2003-10), pages 463-465, XP002406409
- ARNOTT W PATRICK ET AL: "Photoacoustic spectrometer for measuring light absorption by aerosol: instrument description" ATMOS ENVIRON; ATMOSPHERIC ENVIRONMENT 1999 ELSEVIER SCIENCE LTD, EXETER, ENGL, vol. 33, no. 17, 22 September 1997 (1997-09-22), pages 2845-2852, XP002406410

## Description

This invention relates to the field of detection of nitrogen-containing trace-gases, and in particular to a detector unit that uses a converter that converts nitrogen-containing compounds into nitrogen dioxide, and a low cost and compact nitrogen dioxide (NO₂) detector comprising a blue semiconductor laser and quartz-enhanced photo-acoustic sensor.

US Patent 6,612,306 "RESPIRATORY NITRIC OXIDE METER", issued 2 September 2003 to James R. Mault, teaches a respiratory gas meter for detecting gas components of respiratory gas flowing along a flow path. As detailed in this reference, endogenous production of nitric oxide (NO) is increased in patients with asthma and other inflammatory lung diseases, as well as in patients with reactive airways disease. Mault cites USP 5,922,610 "SYSTEM TO BE USED FOR THE DETERMINATION OF NO LEVELS IN EXHALED AIR AND DIAGNOSTIC METHODS FOR DISORDERS RELATED TO ABNORMAL NO LEVELS", issued 13 July 1999 to Alving et al., incorporated by reference herein, for teaching the use of nitric oxide measurements in the diagnosis of inflammatory conditions of the airways, such as allergic asthma and rhinitis, in respiratory tract infections in humans and Kartagener's syndrome, as well as gastric disturbances. Other uses of nitric oxide detection for medical diagnoses are also cited by Mault.

In addition to detecting nitric oxide for medical diagnoses, detectors for measuring the concentrations of various nitrogen oxides, generally referred to as NOx are used to detect environmental concentrations, including vehicle emissions, and for industrial process control. Nitric oxide detectors may also be used to detect explosive material, which is an area of increased security concerns.

US Patent 6,612,306 (Mault) discloses a variety of techniques for detecting nitric oxide, including the detection of fluorescence from the gas induced by radiation, the detection of resonance changes on a micromechanical structure, and the detection of chemiluminescence when ozone is introduced in the airflow.

US Patent 6,160,255 "LASER-BASED PHOTOACOUSTIC SENSOR AND METHOD FOR TRACE DETECTION AND DIFFERENTIATION OF ATMOSPHERIC NO AND NO₂", issued 12 December 2002 to Rosario C. Sausa teaches a dual-laser photoacoustic sensor that excites the air with pulsed, tunable lasers around 227nm and 454nm, and then detects the acoustic effects produced by the heat released from the excited nitric oxide and nitrogen dioxide. The excitation is caused by the nitric oxide absorbing the UV radiation around 227nm, and the nitrogen dioxide absorbing the visible radiation around 454nm.

Besides absorptions in UV, nitric oxide has absorption features in the mid-infrared spectrum. However, the cost of components in the UV and mid-infrared range are orders of magnitude more expensive than their visible-range counterparts, and thus the cost of components for photoacoustic sensing of nitric oxide does not currently provide for a low-cost nitric oxide detector. In USP 6,160,255 (Sausa), a doubling crystal and wavelength compensator are selectively enabled via an arrangement of mirrors to derive the 227nm laser from the 454nm laser. In converting the 454 nm radiation into 227 nm radiation less than 1% of the power remains, which results in a significant reduction in detection sensitivity in the UV. On the other hand, because components in the visible range continue to be developed for high-volume applications, such as optical storage devices (CDs, DVDs) and lighting devices, the cost of these visible-range components continues to decrease.

US 2004/0133116 A1 describes the measurement of nitrogen monoxide in exhaled air by means of a gas sensor being a semi-conducting metal oxide gas sensor for the determination of nitrogen oxides, wherein the NO gas is converted into NO₂ gas in a converter.

If a low-cost nitric oxide detector were available, each physician's office could be equipped with a diagnostic tool that will facilitate the detection and diagnosis of pulmonary and other physiological conditions, and asthmatic patients could be provided with a monitoring device for home use. A low cost NOx sensor could also be used for permanent/continuous exhaust-gas monitoring in cars and environmental-air quality monitoring. Similarly, low-cost nitric oxide detectors could be provided to security personnel at office buildings, train terminals, airports, and other potential terrorist targets.

It is an object of this invention to provide a low-cost, compact and highly sensitive detector for detection of nitrogen-containing gases. It is a further object of this invention to provide a detector that uses photoacoustic techniques. The object of the invention is set forth by a detector of claim 1 and a method of claim 7.

These objects, and others, are achieved in particular by a system that uses a semiconductor laser or light emitting diode emitting in the blue-violet-green wavelength range and a narrow bandwidth photo-acoustic sensor with a resonant pickup unit. Nitrogen dioxide is directly detected by its absorption in the blue-violet wavelength range while other nitrogen-containing compounds are chemically converted into nitrogen dioxide before photosensitive sensing of the amount of nitrogen dioxide produced. A surface chemical oxidation unit is preferably used to convert nitric oxide to nitrogen dioxide, using, for example potassium permanganate (KMnO₄) filter, or a platinum (Pt) catalyst unit.

The invention is explained in further detail, and by way of example, with reference to the accompanying drawings wherein:
FIG. 1 is a schematic view of an example photo-acoustic nitrogen-dioxide detector in accordance with this invention.
FIG. 2A illustrates an example block diagram of a detection unit in accordance with this invention suitable for detection of a nitrogen-containing compound in a gas mixture.
FIG. 2B illustrates an example block diagram of a nitric oxide detection unit in accordance with this invention incorporating a surface chemical conversion unit.
FIG. 2C illustrates an example block diagram of a NOx detection unit in accordance with this invention incorporating an ozone based conversion unit.
FIG. 2D illustrates an example block diagram of a NOx detection unit in accordance with this invention incorporating a catalytic conversion unit.
FIG. 2E illustrates an example block diagram of a nitrogen compound detection unit in accordance with this invention incorporating a scrubber unit and a conversion unit.
FIG. 3A illustrates an example block diagram of a photo-acoustic sensor for use in a nitric oxide breath tester incorporating a scrubber and conversion unit in accordance with this invention.
FIG. 3B illustrates an example block diagram of a photo-acoustic sensor for use in a nitric oxide breath tester incorporating a conversion unit and applying a differential measurement procedure in accordance with this invention.
FIG. 3C illustrates an example block diagram of a photo-acoustic sensor for use in a breath tester enabling the measurement of nitric oxide and one or more other gases in the exhaled breath in accordance with this invention.
FIG. 4 illustrates a photo-acoustic sensor unit according to this invention suitable for the measurement of several gases.

Throughout the drawings, the same reference numeral refers to the same element, or an element that performs substantially the same function. The drawings are included for illustrative purposes and are not intended to limit the scope of the invention.

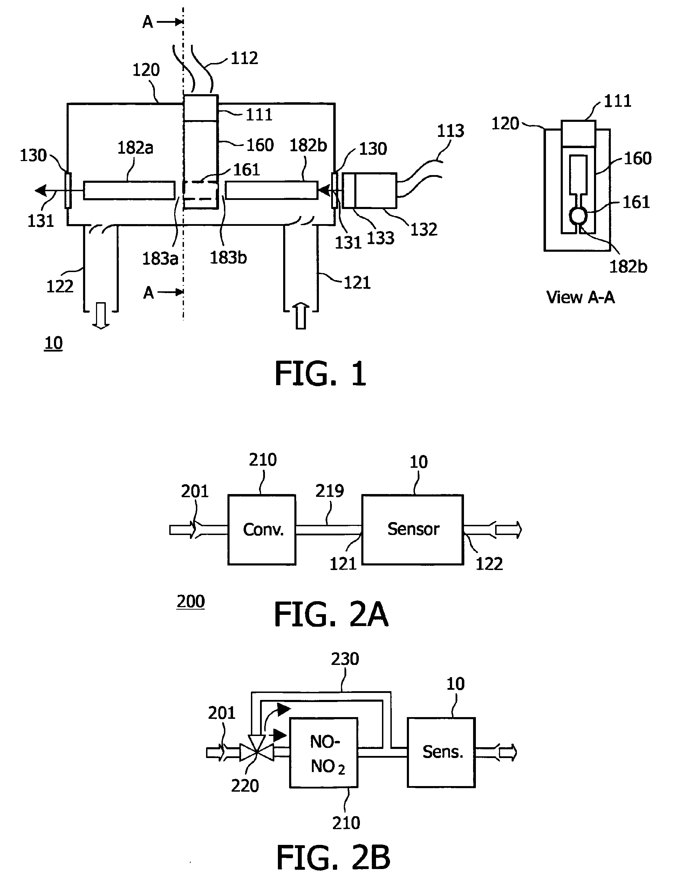

FIG. 1 shows schematically an example photoacoustic nitrogen dioxide sensor 10 in accordance with this invention. The acoustic sensor 10 comprises a piezoelectric sensing unit 111 in the form of a resonant tuning-fork 160 and cylindrical acoustic resonators 182a, 182b. The resonators 182 have open ends to allow gas in the cell 120 to enter the tubes. If necessary, part of the gas cell volume can be filled with solid material to guide the gas flow more efficiently through the resonators. Preferably, the tuning fork has a high quality factor and narrow bandwidth to obtain a high detection sensitivity for nitrogen dioxide and to reduce the sensitivity to acoustic background noise. The acoustic signal enhancing tubes 182a, 182b are positioned as close as possible to the tuning fork 160 so that the spacings 183a, 183b are small and a substantially continuous cavity resonator is formed.

European Patent EP 185 9252 "PHOTOACOUSTIC SPECTROSCOPY DETECTOR AND SYSTEM", filed 4 March 2005 for Hans van Kesteren, discloses a technique for detecting acoustic signals generated in a photoacoustic spectroscopy system through absorption of light that includes a sensing unit that exhibits structural resonance at or near a frequency of the acoustic signals. The sensing unit forms at least part of a cavity resonator that is arranged to enable a formation of standing pressure waves inside the cavity resonator at a cavity resonance frequency substantially coinciding with a structural resonance frequency of the sensing unit. WO 03/104767, "QUARTZ-ENHANCED PHOTOACOUSTIC SPECTROSCOPY", filed 10 June 2003 for Anatoliy A. Kosterev, discloses the fundamental techniques for resonance-enhanced detection of photoacoustic signals.

The sensor 10 incorporates the principles of application FR050029 by choosing the radius of the cylindrical cavity 161 between the prongs of the tuning-fork 160 to be the same as the radii of the tube-shaped signal enhancing means 182a, 182b. The acoustic resonator has a resonance frequency close to or coinciding with the resonance frequency of the tuning fork so both the structural resonance of the tuning fork and the cavity resonance help in obtaining a high photo-acoustic detection sensitivity.

The tuning-fork 160 is provided with electrodes on the various tuning-fork surfaces, using techniques commonly known to those skilled in the field of quartz tuning fork resonators, so as to provide an electric signal that corresponds to the symmetric vibration of the prongs moving in opposite directions. Wires 112 are attached to the tuning fork electrodes to provide the signal from the tuning-fork sensing element to detection electronics (not illustrated).

The tuning-fork pickup element and acoustic resonator are incorporated in a gas cell 120 with gas inlet 121 and gas outlet 122. The gas mixture flows through the gas cell 120 and sensing unit 111. Windows 130 are incorporated in the gas cell 120 to enable a laser beam 131 to pass through the acoustic resonator and between the tuning fork prongs. The laser light with a wavelength in the 370 - 480 nm wavelength range is provided by a small semiconductor laser in a laser module 132. The divergent light form the laser module 132 is collimated by a lens or lens system 133. To obtain a compact sensor, a GRIN (Gradient Index) lens can be used for this purpose.

During operation, the current fed to the laser by wires 113 is modulated to obtain a modulated laser beam at a frequency corresponding to the resonance frequency of the sensing unit, such as an integer multiple (1x, 2x, etc.) or sub-multiple (1/2x, 1/3x, etc.) of the resonance frequency. In the referenced patent application, WO 03/104767, an embodiment is described wherein the wavelength of an infrared laser is modulated at half the resonant frequency of the sensing unit, so as to avoid a direct excitation of the tuning fork by the laser beam. In a preferred embodiment of this invention, however, because low-cost blue semiconductor lasers are more easily power modulated than wavelength modulated and the absorption wavelength range of nitrogen dioxide is broader than the wavelength range available from a blue semiconductor laser, a power modulation of the laser beam, at a frequency equal to the resonant frequency is preferred. In such an embodiment, the beamwidth of the laser inside the tuning fork and acoustic resonator is sufficiently controlled, or other methods employed, to avoid direct excitation of the tuning fork. The shorter wavelength of blue radiation compared to infrared radiation as well as the availablity of high-quality optical components in the visible wavelength range make it easier to obtain these small beamwidths in the blue wavelength range than in the infrared wavelenth range. Laser modulation at other multiples or sub-multiples of the resonant frequency may also be used, albeit with a possible loss of efficiency.

During absorption by the nitrogen dioxide, heat is dissipated in the gas mixture and pressure waves are generated that are amplified by the acoustic resoantor and picked up by the tuning fork. Amplification and phase-sensitive detection of the tuning fork response communicated by the wires 112, using techniques common in the art of photoacoustic detection, provides a result that corresponds to the nitrogen dioxide concentration.

Nitrogen dioxide has a broad absorption band in the visible-light spectrum. As such, because of the greater production quantities of semiconductor lasers and optical components that operate in the visible spectrum, the cost of providing the visible-light sensor 10 can be expected to be substantially less than the cost of an ultraviolet-light or infrared sensors as taught in for instance USP 6,160,255 (Sausa, above). Quartz tuning fork sensor elements can also be produced at low cost because this technology is similar to the technology for making tuning-fork frequency reference crystals. Semiconductor lasers and quartz tuning forks have millimeter size dimensions and low power consumption, which enables the integration of all components into a very compact and low power device, suitable for portable applications.

FIG. 2A shows a detector unit 200 for the detection of one or more nitrogen-containing compounds in a gas mixture. The detector includes a converter 210 that is configured to convert one or more nitrogen-containing compound(s) in the incoming gas mixture 201 to nitrogen dioxide (NO₂) molecules. The converted gas stream 219 is provided to a photoacoustic sensor 10 via inlet port 121 and discharged via the output port 122. The photoacoustic sensor 10 is configured to detect the presence of nitrogen dioxide molecules in the converted air 219.

The converter 210 can use any of a variety of techniques that are well known in the art to convert nitrogen-containing compounds to nitrogen dioxide. Examples of nitrogen-containing compounds are nitric oxide (NO), ammonia (NH₃), and amines. Nitric oxide can be converted into nitrogen dioxide by an oxidation step while ammonia can be converted by, for example, the Oswald process. In the Oswald process, NH₃ is converted into NO₂ by a Pt catalytic reaction and subsequent reaction with oxygen.

A number of example configurations for the detector 200 are provided in FIGs. 2B-2E, although one of ordinary skill in the art will recognize that the invention is not limited to these examples.

FIG. 2B shows an example NO detection unit. The incoming gas mixture 201 is led via a three way valve 220 to a NO to NO₂ conversion unit 210. Preferably, a surface-oxidation device is used, to provide a low cost and compact design. An example of a surface-chemical oxidation device is provided by B. Fruhberger et al. in "Detection and quantification of nitric oxide in human breath using a semiconducting oxide based chemiresistive microsensor," Sensors and Actuators B: Chemical, 76(1-3):226-34, 2001, wherein an alumina supported permanganate (KMnO₄) filter provides a surface-chemical oxidation of nitric oxide into nitric dioxide. The sum of the NO₂ present in the incoming gas stream and the NO₂ generated in the conversion unit is measured in the photo-acoustic sensor 10. To compensate for the presence of NO₂ in the incoming gas stream, a reference measurement can be performed without the converter by leading the incoming gas mixture 201 via the three way valve 220 through the bypass 230 to the sensor 10.

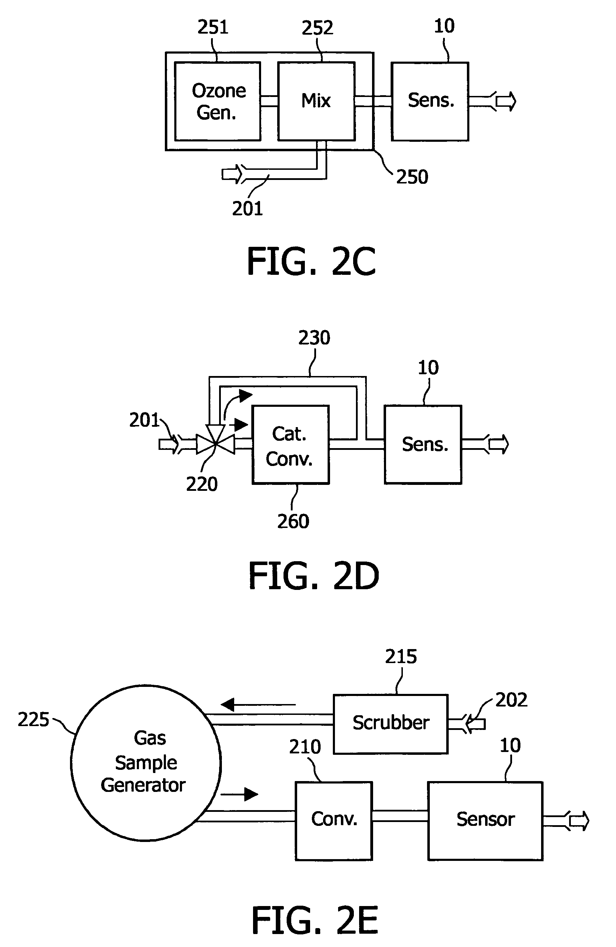

FIG. 2C illustrates a configuration to measure the total NOx concentration i.e. the sum of NO and NO₂ in a gas stream 201. An ozone based converter 250 converts the nitric oxide present in the gas stream 201 into NO₂ which can be detected together with the NO₂ already present in the gas stream. The converter consists of an ozone generator 251 and a mixing chamber 252 in which the ozone reacts with the nitric oxide present in the gas mixture. Ozone can be generated from ambient air by a variety of techniques known to the art.

Alternatively, as illustrated in FIG. 2D, a platinum (Pt) catalytic converter can be used to cause the oxidation of nitric oxide to nitrogen dioxide. This alternative requires that the catalyst be heated to 300° C, but avoids the need to regularly replace the permanganate filter of FIG. 2B. The incoming gas stream 201 from, for instance, the exhaust of a car is led via the catalytic converter 260 to the photo-acoustic sensor 10. If it is necessary to discriminate between NO and NO₂ in the incoming gas mixture, a reference measurement for obtaining the NO₂ concentration can be done by reducing the catalyst temperature below the conversion temperature. Another faster approach is to apply a three way valve 220 and a bypass 230, as illustrated.

FIG. 2E illustrates a block diagram of an example configuration incorporating a scrubber 215 and a converter 210. This configuration can be applied for monitoring the generation of nitrogen-containing gas compounds in an enclosed environment 225. The scrubber removes one or several nitrogen-containing compounds from the incoming gas mixture 202. The scrubber should remove at least nitrogen dioxide, compounds that produce nitrogen dioxide from the incoming gas mixture when led through the converter, and the components that are generated in the compartment 225. The advantage of combining a scrubber and a converter is that a good specificity for the detection of specific nitrogen-containing compounds can be obtained with relatively simple scrubber and converter compositions.

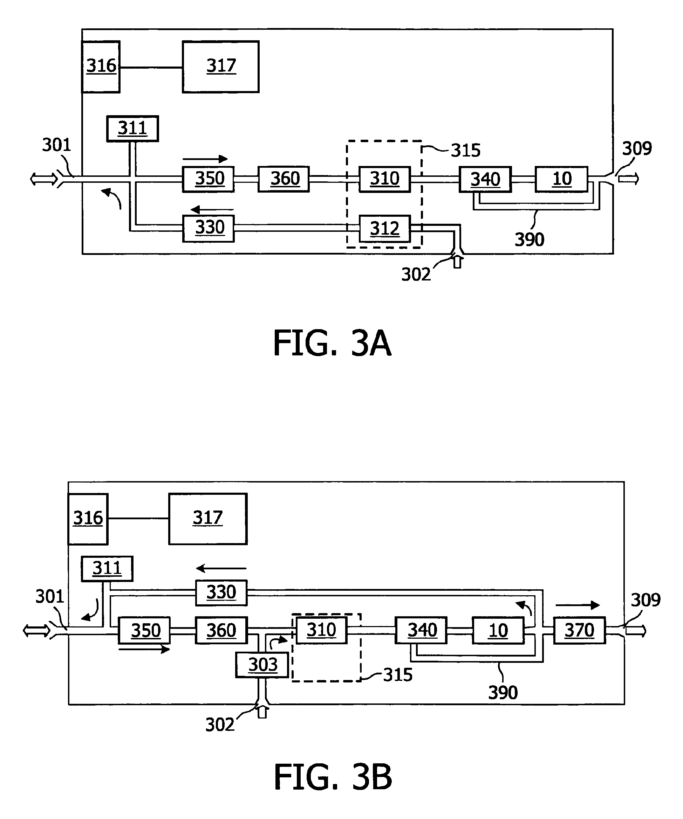

FIG. 3A shows a block diagram of a breath tester suitable for the detection of nitric oxide and based on a scrubber 312, a converter unit 310, and a miniature photo-acoustic sensor 10. During inhalation through the mouthpiece 301, ambient air is led through the inlet 302, scrubber 312, and one-way valve 330. The scrubber removes the NO and NO₂ from the inhaled air. During exhalation, through the mouthpiece 301, the air is guided via one-way valve 350 and flow restrictor 360 to the converter unit 310. Flow restrictor 340 causes a part of the flow to enter the photo-acoustic sensor 10, and another part to enter the bypass 390, and the exhaled air leaves the breath tester via exit port 309.

The scrubber 312 preferably removes the NO and NO₂ from the inhaled air, while the converter 310 is preferably based on a surface chemical conversion of nitric oxide into nitrogen dioxide. The scrubber 312 and converter 310 are part of a replaceable unit 315. Because the inhaled air is free of nitric oxide, all the nitric oxide present in the exhaled gas stream is generated in the body that provides the exhaled air. Therefore, the amount of nitrogen dioxide measured in the sensor 10 directly corresponds to the amount of nitric oxide generated in the body.

A pressure sensor 311 is also provided to monitor exhalation and inhalation. During exhalation, the flow is preferably restricted by 360 to a fixed flow rate that conforms to the generally accepted measuring conditions for nitric oxide measurements in exhaled air. Electronics for signal detection, pressure monitoring, laser power control, etc, are illustrated in block 317. The results of the breath test can be presented to the physician or patient via a user interface 316.

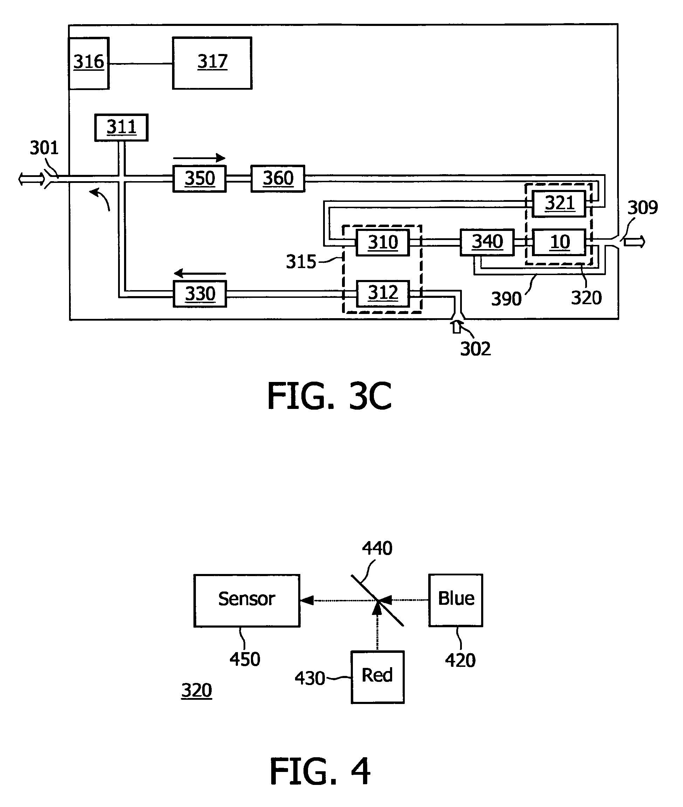

FIGs. 3B and 3C illustrate alternative embodiments of an exhaled breath tester. The blocks similar to those used in FIG. 3A are denoted by the same number.

The exhaled breath tester in FIG. 3B incorporates a replaceable NO to NO₂ converter unit 315 but does not include a scrubber unit. In the embodiment of FIG. 3B, the inhaled air passes through inlet port 302, one-way valve 303, and converter 310. During this stage, the sum of the nitrogen dioxide produced in the converter and the nitrogen dioxide already present in the ambient air is detected by the photo-acoustic sensor 10. As in FIG. 3A, the flow through the detector can be restricted by flow restrictor 340, with the excess of the inhaled air going though bypass 390. After passing another one-way valve 330, the air enters the mouth through the mouthpiece 301; one-way valve 370 prevents the intake of air from the exit port 309.

The exhaled air in FIG. 3B follows a similar path as described for FIG. 3A. The nitric oxide produced in the body can be derived from the difference of NO₂ concentration measured by the sensor unit 10 in the inhaled and exhaled gas mixture.

FIG. 3C shows a block diagram of an exhaled breath tester that measures nitric oxide in combination with one or several other gases in the exhaled breath. In particular, O₂, CO₂, and H₂O are present in high concentrations and can be measured relatively simply with various types of sensors. Measuring the exhalation profile of O₂, CO₂ and/or H₂O and linking this profile to the exhalation profile of nitric oxide provides information that facilitates a better diagnosis of a specific airway disease. It also enables sampling of the NO concentration in the exhaled air stemming from a specific part of the airways or lungs. A measurement unit for measuring O₂, CO₂ or H₂O is denoted by 321 in fig 3C. Oxygen and water have absorption features in the visible range. This makes it attractive to combine a nitrogen dioxide and for instance oxygen sensor into one photo-acoustic module, as illustrated by the dashed combination 320 of sensors 10 and 321.

FIG. 4 illustrates a block diagram of an example combined nitrogen dioxide and oxygen photo-acoustic module for use in the combination 320. Oxygen shows an absorption feature around a wavelength of 760 nm, where semiconductor lasers are available. In the photo-acoustic sensor module 320, a dichroic mirror 440 guides both the laser beam from the red laser unit 430 and the laser beam from the blue laser unit 420 into the photo-acoustic sensor 450, such as the sensor 10 in FIG. 1. The components present in the photo-acoustic sensor 10, such as the tuning fork, acoustic resonator, and windows, are not wavelength specific within the visible wavelength range, and therefore operation at two wavelengths is feasible. During operation, the two lasers can be powered alternatively for short periods of time during the exhalation time, to obtain both the oxygen and nitrogen dioxide exhalation profile.

## Claims

1. A detector (200) for detecting one or more nitrogen-containing compounds in a gas mixture comprising:
a converter (210) that is configured to convert at least one nitrogen-containing compound in the gas mixture into nitrogen dioxide, and
a photo-acoustic sensor (10), operably coupled to the converter (210), that is configured to detect the nitrogen dioxide provided by the converter (210), and includes:
an acoustic pickup unit (111-160) that exhibits a structural resonance at a resonant frequency, and
a light source (132) that is configured to emit light at a wavelength corresponding to an absorption of nitrogen dioxide in a visible wavelength range, and with a modulation frequency corresponding to the resonant frequency, wherein the acoustic pickup unit (111-160) includes a piezoelectric sensing unit (111) that includes a resonant tuning-fork (160) and one or more cylindrical acoustic resonators (182a-b), wherein the tuning-fork (160) is configured to include a cylindrical cavity (161) that has a radius that is substantially equal to a radius of the one or more cylindrical acoustic resonators (182a-b).

2. The detector (200) of claim 1, wherein
the light source (132) includes at least one of: a semiconductor laser and a light emitting diode.

3. The detector (200) of Claim 1, further including
a scrubber (215) that is configured to remove one or more nitrogen-containing compounds from an input that is used to generate the gas mixture.

4. The detector (200) of Claim 1, further including
a pressure sensor (311) that is configured to determine an amount of pressure associated with the gas mixture.

5. The detector (200) of claim 1, wherein
the sensor is configured to detect (321) at least one other gas in the gas mixture.
the light source (132) is further configured to emit light at a wavelength corresponding to an absorption of the at least one other gas in a visible wavelength range.

6. The detector (200) of Claim 1, wherein
the modulation frequency is substantially equal to one of: the resonant frequency, a multiple of the resonant frequency, and a sub-multiple of the resonant frequency.

7. A method of detecting one or more nitrogen-containing compounds in a gas mixture, the method comprising:
converting (210) at least one nitrogen-containing compound in the gas mixture into nitrogen dioxide, and
detecting (10) the nitrogen dioxide by
exposing (120) the gas mixture to light (131) at a wavelength corresponding to an absorption of nitrogen dioxide in a visible wavelength range within a resonant cavity (182a-b, 161), wherein the resonant cavity comprises a cylindrical cavity (161) and one or more cylindrical acoustic resonators (182a-b);
modulating (113) the light at a modulation frequency corresponding to a resonant frequency of the resonant cavity (182a-b, 161), and
detecting (111, 160) vibrations that are produced within the resonant cavity (161) due to the absorption with an acoustic pickup unit (111-160) which includes a piezoelectric sensing unit (111) that includes a resonant tuning-fork (160) and the one or more cylindrical acoustic resonators (182a-b), wherein the tuning-fork (160) is configured to include the cylindrical cavity (161) that has a radius that is substantially equal to a radius of the one or more cylindrical acoustic resonators (182a-b).

8. The method of claim 7, further including
scrubbing (215) an input that is used to generate the gas mixture, to remove nitrogen-containing compounds in the input.

9. The method of claims 7, wherein
converting (210) the at least one nitrogen-containing compound includes at least one of:
oxidation,
catalytic conversion (260), and
ozone generation (251).

## Patentansprüche

1. Detektor (200) zum Detektieren einer oder mehrerer stickstoffhaltiger Verbindungen in einem Gasgemisch mit:
einem Umwandler (210), der so ausgelegt ist, dass er zumindest eine stickstoffhaltige Verbindung in dem Gasgemisch in Stickstoffdioxid umwandelt, und
einem fotoakustischen Sensor (10), der von der Funktion her mit dem Umwandler (210) verbunden und so ausgelegt ist, dass er das von dem Umwandler (210) gelieferte Stickstoffdioxid detektiert und Folgendes umfasst:
einen akustischen Impulsgeber (111-160), der eine Strukturresonanz mit einer Resonanzfrequenz besitzt, und
eine Lichtquelle (132), die so ausgelegt ist, dass sie Licht mit einer Wellenlänge, die der Absorption von Stickstoffdioxid in einem sichtbaren Wellenlängenspektrum entspricht, und mit einer Modulationsfrequenz, die der Resonanzfrequenz entspricht, abstrahlt, wobei der akustische Impulsgeber (111-160) einen piezoelektrischen Sensor (111) umfasst, der eine Resonanz-Stimmgabel (160) und einen oder mehrere zylindrische, akustische Resonatoren (182a-b) umfasst, wobei die Stimmgabel (160) so ausgelegt ist, dass sie einen zylindrischen Hohlraum (161) enthält, dessen Radius im Wesentlichen dem Radius des einen oder der mehreren zylindrischen akustischen Resonatoren (182a-b) entspricht.

2. Detektor (200) nach Anspruch 1, wobei
die Lichtquelle (132) zumindest entweder einen Halbleiterlaser oder eine Licht emittierende Diode umfasst.

3. Detektor (200) nach Anspruch 1, der ferner Folgendes umfasst:
einen Gaswäscher (215), der so ausgelegt ist, dass er eine oder mehrere stickstoffhaltige Verbindungen aus einem eingespeisten Gas entfernt, das zur Erzeugung des Gasgemisches verwendet wird.

4. Detektor (200) nach Anspruch 1, der ferner Folgendes umfasst:
einen Drucksensor (311), der so ausgelegt ist, dass er einen dem Gasgemisch zugeordneten Druck ermittelt.

5. Detektor (200) nach Anspruch 1, wobei
der Sensor (321) so ausgelegt ist, dass er mindestens ein weiteres Gas in dem Gasgemisch detektiert,
die Lichtquelle (132) ferner so ausgelegt ist, dass sie Licht mit einer Wellenlänge abstrahlt, die der Absorption von zumindest einem weiteren Gas in einem sichtbaren Wellenlängenspektrum entspricht.

6. Detektor (200) nach Anspruch 1, wobei
die Modulationsfrequenz im Wesentlichen entweder der Resonanzfrequenz, einem Vielfachen der Resonanzfrequenz oder einem Bruchteil der Resonanzfrequenz entspricht.

7. Verfahren zum Detektieren einer oder mehrerer stickstoffhaltiger Verbindungen in einem Gasgemisch, wobei das Verfahren Folgendes umfasst:
Umwandeln (210) zumindest einer stickstoffhaltigen Verbindung in dem Gasgemisch in Stickstoffdioxid und
Detektieren (10) des Stickstoffdioxids durch
Belichten (120) des Gasgemischs mit Licht (131) mit einer Wellenlänge, die der Absorption von Stickstoffdioxid in einem sichtbaren Wellenlängenspektrum in einem Resonanzhohlraum (182a-b, 161) entspricht, wobei der Resonanzhohlraum einen zylindrischen Hohlraum (161) und einen oder mehrere zylindrische akustische Resonatoren (182a-b) umfasst,
Modulieren (113) des Lichts mit einer Modulationsfrequenz, die der Resonanzfrequenz des Resonanzhohlraums (182a-b, 161) entspricht, und
Detektieren (111, 160) von Vibrationen, die in dem Resonanzhohlraum (161) aufgrund der Absorption mit einem akustischen Impulsgeber (111-160) erzeugt werden, der einen piezoelektrischen Sensor (111) umfasst, der eine Resonanzstimmgabel (160) und den einen oder mehrere zylindrische akustische Resonatoren (182a-b) enthält, wobei die Stimmgabel (160) so ausgelegt ist, dass sie den zylindrischen Hohlraum (161) enthält, der einen Radius aufweist, der im Wesentlichen dem Radius des einen oder der mehreren zylindrischen akustischen Resonatoren (182a-b) entspricht.

8. Verfahren nach Anspruch 7, das ferner Folgendes umfasst:
Waschen (215) eines eingespeisten Gases, das zur Erzeugung des Gasgemischs verwendet wird, um stickstoffhaltige Verbindungen aus dem eingespeisten Gas zu entfernen.

9. Verfahren nach Anspruch 7, wobei
das Umwandeln (210) der mindestens einen stickstoffhaltigen Verbindung zumindest eine der folgenden Prozesse umfasst:
Oxidation,
katalytische Umwandlung (260), und
Ozonerzeugung (251).

## Revendications

1. Détecteur (200) pour détecter un ou plusieurs composés contenant de l'azote dans un mélange gazeux, comprenant :
un convertisseur (210) qui est configuré pour convertir au moins un composé contenant de l'azote dans le mélange gazeux en dioxyde d'azote, et
un capteur photo-acoustique (10), couplé fonctionnellement au convertisseur (210), qui est configuré pour détecter le dioxyde d'azote fourni par le convertisseur (210), et comprend :
une unité de captation acoustique (111-160) qui présente une résonance structurelle à une fréquence résonante, et
une source lumineuse (132) qui est configurée pour émettre de la lumière à une longueur d'onde correspondant à une absorption de dioxyde d'azote dans une plage de longueurs d'onde visibles, et avec une fréquence de modulation correspondant à la fréquence résonante, dans lequel l'unité de captation acoustique (111-160) comprend une unité de détection piézoélectrique (111) qui comprend un diapason résonant (160) et un ou plusieurs résonateurs acoustiques cylindriques (182a-b), dans lequel le diapason (160) est configuré pour comprendre une cavité cylindrique (161) qui possède un rayon qui est sensiblement égal à un rayon du ou des résonateurs acoustiques cylindriques (182a-b).

2. Détecteur (200) selon la revendication 1, dans lequel
la source lumineuse (132) comprend au moins un élément parmi un laser à semi-conducteur et une diode électroluminescente.

3. Détecteur (200) selon la revendication 1, comprenant en outre
un épurateur (215) qui est configuré pour éliminer un ou plusieurs composés contenant de l'azote à partir d'une entrée qui est utilisée pour générer le mélange gazeux.

4. Détecteur (200) selon la revendication 1, comprenant en outre
un capteur de pression (311) qui est configuré pour déterminer une quantité de pression associée au mélange gazeux.

5. Détecteur (200) selon la revendication 1, dans lequel
le capteur est configuré pour détecter (321) au moins un autre gaz dans le mélange gazeux,
la source lumineuse (132) est en outre configurée pour émettre de la lumière à une longueur d'onde correspondant à une absorption de l'au moins un autre gaz dans une plage de longueurs d'onde visibles.

6. Détecteur (200) selon la revendication 1, dans lequel
la fréquence de modulation est sensiblement égale à un élément parmi la fréquence résonante, un multiple de la fréquence résonante, et un sous-multiple de la fréquence résonante.

7. Procédé de détection d'un ou de plusieurs composés contenant de l'azote dans un mélange gazeux, le procédé comprenant :
la conversion (210) d'au moins un composé contenant de l'azote dans le mélange gazeux en dioxyde d'azote, et
la détection (10) du dioxyde d'azote en
exposant (120) le mélange gazeux à de la lumière (131) à une longueur d'onde correspondant à une absorption de dioxyde d'azote dans une plage de longueurs d'onde visibles à l'intérieur d'une cavité résonante (182a-b, 161), dans lequel la cavité résonante comprend une cavité cylindrique (161) et un ou plusieurs résonateurs acoustiques cylindriques (182a-b) ;
modulant (113) de la lumière à une fréquence de modulation correspondant à une fréquence résonante de la cavité résonante (182a-b, 161), et
détectant (111, 160) de vibrations qui sont produites à l'intérieur de la cavité résonante (161) en raison de l'absorption avec une unité de captation acoustique (111-160) qui comprend une unité de détection piézoélectrique (111) qui comprend un diapason résonant (160) et le ou les résonateurs acoustiques cylindriques (182a-b), dans lequel le diapason (160) est configuré pour comprendre la cavité cylindrique (161) qui possède un rayon qui est sensiblement égal à un rayon du ou des résonateurs acoustiques cylindriques (182a-b).

8. Procédé selon la revendication 7, comprenant en outre
l'épuration (215) d'une entrée qui est utilisée pour générer le mélange gazeux, pour éliminer des composés contenant de l'azote dans l'entrée.

9. Procédé selon la revendication 7, dans lequel
la conversion (210) de l'au moins un composé contenant de l'azote comprend au moins un élément parmi :
une oxydation,
une conversion catalytique (260), et
une génération d'ozone (251).
